# EUROPEAN PATENT APPLICATION

(11) **EP 1 801 207 A1**
(43) Date of publication of application: **27.06.2007**
(21) Application number: 05788187.2
(22) Date of filing: 29.09.2005
(51) Int. Cl.: C12N 15/00, C12Q 1/02, C12Q 1/68, G01N 21/78

(54) **METHOD OF DETECTING INTRACELLULAR MICRONUCLEUS**

(30) Priority: 30.09.2004 JP 2004288924
(71) Applicant: Olympus Corporation, Tokyo 151-0072 (JP); Riken, Wako-shi, Saitama 351-0198 (JP)
(72) Inventor: SUZUKI, Hirobumi, Olympus Intellectual Property Services Co.,Ltd., Hachioji-shi, Tokyo 192-8512 (JP); MIYAWAKI, Atsushi, c/o Riken, Wako-shi, Saitama 351-0198 (JP)
(74) Representative: von Hellfeld, Axel
(86) International application number: PCT/JP2005/018005
(87) International publication number: WO 2006/035895

(57) **Abstract**

The present invention provides a method of detecting a micronucleus in a living cell to be measured, without having to fixate the cell, **characterized by** comprising generating a mother cell by introducing a gene encoding a single or a plurality of nucleus-related proteins and a gene encoding a fluorescence protein into a culture cell, and making then expressed in the cell, subjecting the cell to a treatment for a toxicity test or a mutagenicity test of a test substance, performing a limited excitation which makes a region of the micronucleus emit fluorescence limitedly, and quantitatively measuring an amount of the fluorescence corresponding to a component of the nucleus-related protein.

## Description

### Technical Field

The present invention relates to a method of detecting a micronucleus in a cell.

### Background Art

In the evaluation of safety of a drug, chemical substance, or the like, long-term animal trials using mouse, etc. are generally carried out in order to assess its carcinogenicity. However, since such tests for safety using the animal trials require a great amount of time and cost, mutagenic tests such as reverse mutation test using bacteria and chromosomal aberration test using cultured mammalian cells are usually carried out.

The reverse mutation test that uses bacteria has such characteristics that a mutation that occurred at a gene level can be detected and the positive predictability is relatively high. Therefore, when a result of the test is positive, the substance is considered to be carcinogenic at a high probability.

However, there are a great number of carcinogenic substances that cannot be detected by the test. For this reason, at present, the most of the test organizations carry out, in combination with the gene mutation test as its complement test, the chromosomal aberration test that uses cultured mammalian cells to detect damage to genome, which is significantly related to the development and progress of a tumor.

Induction of chromosomal structural abnormality and/or numerical abnormality is used as an index in the chromosomal aberration test. And, chromosomal aberration test has been successfully used to detect some carcinogenic substances such as benzene and diethylstilbestrol that were judged to be negative in the reverse mutation test.

However, it requires a high-grade skill and time for the chromosomal analysis and chromosomal aberration test, and therefore these tests entail a drawback of time consumption. Further, the numerical abnormality is limited to the detection of a polyploidy in which chromosomes increase by an integral multiple of the fundamental number thereof. Therefore, such an aneuploidy as that of a human inheritable disease in which the number of chromosomes increases or decreases by one to several cannot be detected, and thus there is a limitation in usage.

Under these circumstances, validations of *in vitro* micronucleus tests are conducted not only in Japan but also in other countries as an alternative to the chromosome abnormality test, in order to quickly detect a chromosome abnormality inducing substance.

Micronuclei are small nuclei having a size of less than 1/3 of the diameter of main nuclei (also referred to as macronuclei) remaining in cytoplasm of daughter cell after cell division. It is considered that micronuclei are formed not only due to a structural abnormality of a chromosome but also a chromosomal nondisjunction caused by the inhibition of cell division, which results in aneuploidy. Since the observation of micronuclei in a cell is far more easy than the chromosomal analysis, it is considered that the in vitro micronucleus test is more suitable for the routine test than the chromosome abnormality test.

However, the micronucleus test is standardized conventionally as an *in vivo* test that uses myelogenic erythrocyte of a mouse or the like, and there is a problem unsolved that its test protocol has not been established as an alternative to the chromosome abnormality test.

Under a commission from the Ministry of Labor of Japan, five facilities carried out researches on an *in vitro* micronucleus test that uses a cell of cell strain originated from the lung of Chinese hamster (CHL/IU) from 1987 as "Investigation regarding Accuracy Management Technique of the *in vitro* Micronucleus Test" for the purpose of complementing the mutagenesis test and enhancing the accuracy of the screening of the carcinogenicity of a chemical substance. In the researches, the effectiveness of the *in vitro* micronucleus test was examined, and the standardization of the protocol was fostered. As a result, "Standards for Nucleus Test that Uses Cultured Mammalian Cell" (Secondary Draft) was formed.

The results of the *in vitro* micronucleus tests carried out on 66 substances during the researches indicated a high concordance rate of 88.7% with regard to the results of the *in vitro* chromosome abnormality tests, and this fact suggests that these tests are useful as an alternative method. (See Matsushima, T. et al., "Validation study of the in vitro micronucleus test in a Chinese hamster lung cell line (CHL/IU).", Mutagenesis, 1999, Vol. 14, p. 569-580.) Thus, in recent years, the micronucleus test is becoming more important as a highly versatile and safe test with a high throughput and quantitative property, which can evaluate more chemical substances, and has the third significance, in which the mutagenic test is carried out in vivo, in addition to the reverse mutation test that uses bacteria and the chromosome abnormality test that uses cultured mammalian cells having a higher sensitivity.

At present, in Japan, a "micronucleus test that uses a rodent" and the above-described "in vitro micronucleus test that uses cultured cell" are carried out as the mutagenic tests based on the "law regarding the regulations on the examination, preparation, etc of chemical substances".

For example, the technical contents of the "micronucleus test that uses a rodent" are as follow.

### (1) Micronucleus test using a rodent

### "Object and application range of micronucleus test"

The genetic toxicity of a substance to be tested is detected by a relatively simple and short-term test, and based on the results of the detection, the carcinogenicity and genetic influence on the next generation are predicted.

There are various types of methods in the mutagenic test. Of these, the "reverse mutation test that uses bacteria" is carried out as a test indicating the gene mutation inducibility, and the "chromosome abnormality test that uses cultured mammalian cells" is carried out as a test indicating the chromosome abnormality inducibility. When a positive result is obtained in at least one of these tests, the "micronucleus test that uses a rodent" is to be carried out.

### "Animal and Observation Cell"

A young mature rodent is used, and juvenile erythrocyte of bone marrow or peripheral blood is to be observed. In general, a mouse or rat is used. In the case of rat, the appearance of pseudo-micronucleus due to the granulation of mast cell should be carefully watched when the bone marrow is used, whereas the elimination of erythrocyte containing micronucleus in the spleen should be watched when the peripheral blood is used. In this manner, the best possible observation method should be selected.

### "Sex and Number of Samples of the Animal"

5 or more samples per each sex should be selected as one group.

If there is no significant difference observed in terms of toxicity between sexes, a group of only one sex (5 or more samples) should be used.

### "Preparation of Substances to be Tested"

When the test substance is a solid material, it should be solved in an appropriate solvent or suspended in a medium to prepare the sample. When the substance is a liquid, it may be administered directly or the liquid may be diluted with an appropriate solvent to prepare the sample. When the substance is a gas, it should be diluted with a clean air or the like. If the stability of the sample after the preparation thereof is known, it should be used within its stable period, whereas if the stability is not known, it should be prepared when the sample is needed.

### "Control Group"

As the negative control, a solvent or a medium is assigned, whereas an appropriate conventionally known micronucleus inducing substance is assigned as the positive control.

### "Administration Route"

As the basic rule, the forced oral administration or intraperitoneal injection should be selected, except for the case where there is a scientific reason not to select one of these routes, for example, that a specific exposure route (such as inhalation exposure) is expected.

### "Number of times of Administration"

The number of times of administration should be one or more.

### "Dosage Steps"

The maximum dosage is set to a dosage at which a cell toxicity in bone mallow is observed such as a decrease in number of immature erythrocytes is observed, or a dosage at which some sort of sign of toxicity or over which a lethal case is expected, or a technically administrable upper limit.

In the case where no particular sign of toxicity is observed, the maximum dosage is set to 2,000 mg/kg/day for a single administration or a repetitive administration with an interval of 14 days or less. For a long-term repetitive administration with an internal of more than that, the maximum dosage is set to 1,000 mg/kg/day.

It should be noted when the test substance is a gas, the maximum dosage is set to that create a concentration at which it can be safely exposed.

As an appropriate interval (as the basic rule, a common ratio of 2 is assigned, but it is acceptable when a common ratio of √10 or less), a 3 steps or more of dosage is assigned.

### "Sample Preparation Timing"

### - When bone mallow is used

For the single administration, at least two times of sample preparation timing are set with an appropriate interval of 24 to 48 hours after the administration, and based on this timing, the animal is slaughtered and the bone mallow smear is prepared. Or, when the repetitive administration is carried out, the sample is prepared once in a time period of 18 to 24 hours after the final administration.

### - When peripheral blood is used

For the single administration, at least two times of blood sampling timing are set with an appropriate interval of 36 to 72 hours after the administration, and based on this timing, the sample is prepared. Or, when the repetitive administration is carried out, the sample is prepared once in a time period of 24 to 48 hours after the final administration.

### "Observation"

Before the observation, all of the slide samples including the negative and positive controls are encoded so that the observation is carried out in a situation where the processing conditions are not known.

2,000 or more juvenile erythrocytes are observed per individual, and the frequency of appearance of cells having a micronucleus is obtained.

Further, as an index for the suppression of proliferation of marrow bone cells, the frequency of appearance of juvenile erythrocytes with respect to all the erythrocytes is obtained by observing 200 or more cells per individual when the marrow bone is used, whereas it is obtained by observing 1,000 or more cells when the peripheral blood is used.

### "Indication of Results"

For each individual, the frequency of appearance of cells having micronucleus with respect to the observed juvenile erythrocytes and the frequency of appearance of juvenile erythrocytes with respect to all the erythrocytes are indicated in a format of table, together with the average value for each group.

### "Interpretation of Results"

On the presumption that the test substance is appropriately administered to a sufficient effective amount and expected results are obtained in the negative and positive control groups, the results are interpreted by using an appropriate statistical process including utilization of the background data of the negative control group. It should be noted that if there is no clear difference in result between different sexes, the statistical process may be carried out for the total of the data of both sexes taken together.

In case where it is not judged as negative or positive clearly, it is desirable that the case is retested under different experimental conditions to make the final decision since the statistical significance is not the only one criterion to make the judgment.

### "Evaluation of Results"

When a test substance is interpreted to have a positive result in any of the *in vitro* tests and a negative result in the formal test, the interpretation of the result should be considered, for example, by consulting the information available regarding the in vivo fate thereof.

The following are the technical contents of the "*in vitro* micronucleus test that uses cultured cells".

### (2) In vitro micronucleus test using cultured cells

### "Preparation of Samples"

- Cells in a logarithmic growth phase (3 to 5 days from the start of the culture) are used and the culture medium in the culture flask is removed with an aspirator.
- A small amount of a 0.25%-trypsin solution is added to the culture flask and the surfaces of the cells are washed softly, then removing the solution.
- A trypsin solution is newly added to the flask and the resultant is let stand for about 5 minutes at a temperature of 37°C. When the cells are start to detached from the flask, the cells are removed from the flask with pipetting.
- A centrifuge tube containing a fresh culture solution (1 to 2 times as much of the amount of the 0.25 trypsin solution) is prepared. The cells removed in the previous step are transferred to the tube and are centrifuged at 1000 rpm for 5 minutes.
- The supernatant is removed with an aspirator, and a fresh culture solution is added to prepare a cell suspension having an appropriate density (30 to 50 × 10⁴/ml).
- The number of cells is counted with a hemocytometer.
- A solution having a cell density of 1 × 10⁴/ml is prepared. 5 ml of the solution is plated on a 60-mm culture plate.
- The cells are cultured with a 5%-CO₂ incubator at 37°C.
- After culturing, the culture solution is transferred to a centrifuge tube, and then immediately, 2 ml of 0.25%-trypsin solution is added to the plate. After several minutes, the cells are removed from the plate with pipetting and they are transferred to the respective centrifuge tube.
- After the centrifugation (1,000 rpm, for 5 minutes), the supernatant is discarded. 3 to 5 ml of a 0.075M-KCL solution is added and the resultant is let stand at room temperature for 10 minutes. (Hypotonic treatment)
- The resultant is softly stirred. 0.5 ml of cool fixing solution (cooling ethanol : acetic acid = 3 : 1) prepared immediately before addition. The fixing solution is added thereto to semifixing the resultant.
- Immediately after, the resultant is centrifuged (1,000 rpm, for 5 minutes), the supernatant is discarded and 5 ml of a fresh fixing solution is added, and the resultant is again centrifuged. This operation is repeated 3 times.
- Lastly, the fixing solution is replaced by methanol containing 1%-acetic acid, and the resultant is centrifuged.
- A cell suspension having an appropriate concentration (with which the solution is slightly cloudy) is prepared from the fixing solution of the previous step, and one drop of the suspension is put on a clean slide glass, followed by air-drying. Here, the cell density and the like are checked under an inverted microscope. 2 slides are usually prepared and the number may be increased or decreased in accordance with the state of the cells.
- An acridine orange solution (40 µg/ml, dissolved with PBS) is applied on a slide sample drop by drop. The applied solution is immediately covered with a cover glass. After that, the excessive portion of the dye solution is removed, and the sample is observed under a fluorescence microscope.

### "Observation"

- Acridine orange dye: the sample is observed under a fluorescence microscope equipped with a B-excited illumination system at a magnification of 200 times. Samples stained with Giemsa are observed at a magnification of 400 times.

The objects of the observation should be limited to those cells in which the cytoplasm is preserved in good conditions and the shape of the main nucleus is not irregular.

For each concentration, 2,000 cells are observed, and the frequency of appearance of cells containing micronuclei is counted.

The micronuclei are grouped into the following categories.

MN-1: Mononuclear cells containing a micronucleus having a size of 1/10 or less of that of the main nucleus.

MN-2: Mononuclear cells containing a micronucleus having a size of 1/10 to 1/3 of that of the main nucleus.

MN-3: Mononuclear cells containing a micronucleus having a size of 1/3 to 1/2 of that of the main nucleus.

Mu-MN: Mononuclear cells containing a plurality of micronuclei.

MN-T: A total of mononuclear cells containing a micronucleus (a total of the above 4 items). This is the most important item.

Mu-M: Multinucleated cells (including those strongly damaged)

(Mu+MN: multinucleated cells containing micronucleus, which are, this time, included in Mu-M)

MP: Cells in metaphase

total count: a total of cells counted (measured to achieve 2,000 as a target count this time)
- In an automatic measurement using an imaging site meter, the cytoplasm and nucleus are dyed with different fluorescent materials (for example, the former with FITC and the latter with PI), and the calculation is made on the micronucleus in the region of the cytoplasm as above description.
- Cytochalasin B, which has a function of inhibiting polymerization that forms a filament from actin monomers as it bonds to an end of actin filament, is added to test cells immediately after the start of cell division. Thus, the statistical population for which the appearance frequency is calculated is limited only to the two-nuclei cells when detection. Such a technique better assures that micronuclei are generated always through the cell division. This technique is very popular particularly in the U.S and Europe.

### Disclosure of Invention

### [Problems to be Solved by the Invention]

The above-described conventional technique entails the below-described problems with each of the micronucleus test using rodents and the in vitro micronucleus test that uses cultured cells.

The first problem is that the currently employed staining method entails drawbacks of its complicated procedure and disparity in results among testers, and therefore it is difficult to establish a certain standard among the test facilities. Further, when the automatic measurement is assumed, there are drawbacks in quantitative aspect and reproducibility due to the fact that the nucleus region and cytoplasm region are hard to be distinguished from each other by the conventional staining method.

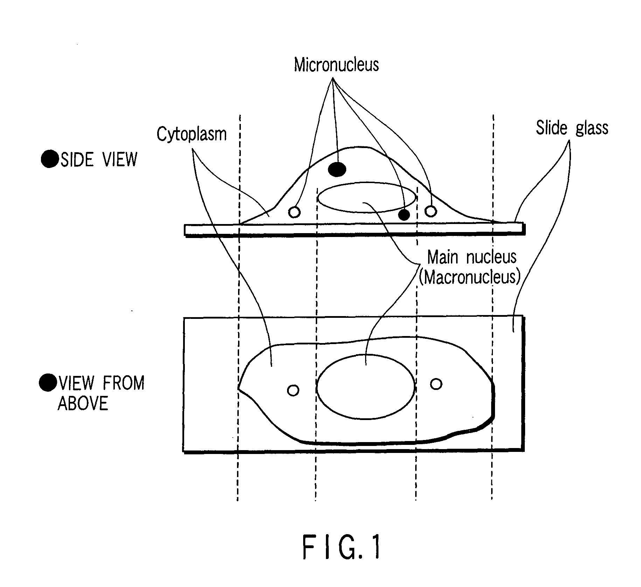

As the second problem, since micronuclei located in the vicinity of the macronucleus region in the cytoplasm are detected as shown in FIG. 1, those micronuclei which are not overlaid with fluorescent materials of the macronucleus in the observation/measurement direction (those of micronuclei which are indicated with open circles) can be detected, but those which are located in an upper region or lower region of the macronucleus or those which are located in a region overlaid with the macronucleus (those of micronuclei which are indicated with closed circles) cannot be detected. It is considered that in the case of mammalian cells, the region of the macronucleus that occupies within the entire observation region of the cell reaches even up to 1/3 to 1/2. For this reason, the number of micronuclei within an entire cell cannot be accurately measured with the conventional method.

The third problem is that there is no guarantee that the addition of a chemical substance that is different from the test substance, such as cytochalasin B, to the test system, is absolutely no influence on the frequency of appearance of micronuclei. Further, in consideration of the presence/absence of interaction between the test substance and cytochalasin B, the addition of such a substance serves to cause a decrease in sensitivity in the detection and quantification of the toxicity and the mutagenicity of the test substance itself.

### [Means for Solving the Problem]

In order to solve the above-described problems, there is provided, according to an aspect of the present invention, a micronucleus detection method which will now be described.
(1) A method of detecting a micronucleus in a living cell to be measured, without having to fixate the cell, the method characterized by comprising: generating a mother cell by introducing a fusion gene encoding a single or a plurality of nucleus-related proteins and a fluorescence protein into a culture cell, and making then expressed in the cell; subjecting the cell to a treatment for a toxicity test or a mutagenicity test of a test substance; performing a limited excitation which makes a region of the micronucleus emit fluorescence limitedly; and quantitatively measuring an amount of the fluorescence corresponding to a component of the nucleus-related protein.
(2) The detection method according to that recited in (1), characterized in that the nucleus-related protein is of a type specific to a nuclear membrane.
(3) The detection method according to that recited in (1), characterized by further comprising: irradiating light while focusing on a region of a macronucleus of the fused protein expressing mother cell, thereby attenuating the fluorescence of only the macronucleus region, and detecting only the fluorescence derived from only the micronucleus in the cell excluding the macronucleus.

### [Advantage of the Invention]

According to the present invention, the drawbacks of the conventional method can be overcome, and the detection of intercellular micronuclei can be carried out easily and quickly at a high quantitative property, high reproducibility and high accuracy. Further, in view of the future market of drug creation, it is expected that it can be applied to tests of many more test compounds and an excellent standardization can be achieved.

### Brief Description of Drawings

[FIG. 1] This figure is a diagram showing the arrangement of micronuclei in the vicinity of the macronucleus in cytoplasm and how they are observed.
[FIG. 2] This figure is a diagram showing the excitation in the measurement of micronuclei and the state of the observation image.

### Best Mode for Carrying Out the Invention

An embodiment of the present invention will now be described, but the invention is not limited to the embodiment.

In this embodiment, a gene in which nucleus-related protein and fluorescence protein are fused is introduced in advance in a cultured mammalian cell such as of the lung of Chinese hamster (CHL), and a cell strain in which the introduced gene is expressed stably in the cell is established. With use of the cell strain as the mother cell, the micronucleus test is carried out as in the case of the conventional technique. After the reaction with the chemical substance and preparation of the fixed sample, the excitation corresponding to the fluorescence protein introduced is directly carried out. Then, the nucleus-related region is measured, and the targets are counted as in the conventional method. Here, the present invention does not require staining procedure utilized in conventional method, but it is possible to detect cell regions from the cell shape by scattered light or from a simple staining such as FITC staining, and count micronuclei in the region.

Here, after the reaction with the chemical substance, the fixed sample is not prepared, and the measurement and calculation of micronuclei is made directly from the culture flask such as a multi-well plate. Here, unlike the test where the conventional end point is set at the measurement point, it is possible to carry out a sequential measurement such as at what point a micronucleus is created during the reaction with the chemical substance, and thus it becomes possible to count micronuclei occurred during the cell division at a high accuracy without using any additional compound such as cytochalasin B.

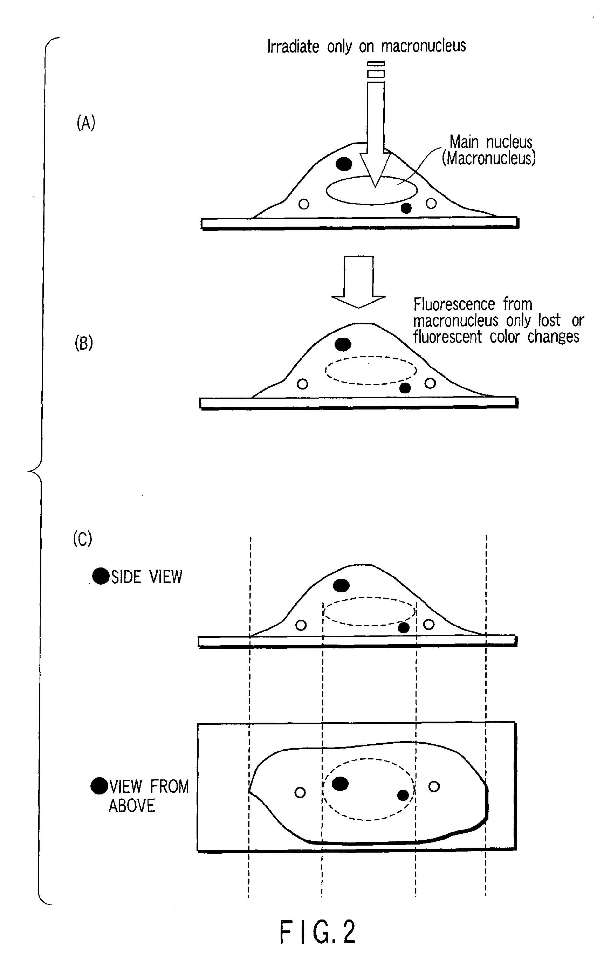

The excitation corresponding to the fluorescence protein described above is carried out by a method shown in FIG. 2. That is, the fluorescence protein used in the present invention has properties of attenuating the fluorescence or changing its fluorescent color when a certain type of light such as visible light is irradiated thereon. When the light irradiation is focused on the region of the macronucleus of the fused protein expressing mother cell during the measurement (FIG. 2A), the fluorescence of only the macronucleus region can be attenuated (FIG. 2B), or the fluorescent color can be changed. Thus, it becomes possible to detect micronuclei which cannot be detected with the conventional method as they are hidden behind the macronucleus (FIG. 2C). In this manner, it is possible to calculate all the micronuclei occurred in the entire cell region and therefore it becomes possible to quantify the toxicity, mutagenicity, etc. caused by a chemical substance at a higher accuracy as compared to the case of the conventional technique.

Further, it is preferable that the mother cell should be established to target the nuclear membrane component as the nucleus-related protein, and the micronucleus test should be carried out as in the case of the conventional technique. In this case, it is possible to detect the nucleus region as the fluorescence amount in proportional to the area of the nucleus with less unevenness as compared to the nucleus region detected with reference to a nucleic acid component or the like. Therefore, the measurement and calculation of micronuclei can be carried out at higher reproducibility and better quantitative property.

The above-described embodiment indicates that the present invention exhibits the following advantageous effects.
(1) The conventional micronucleus test requires a further fluorescent staining after the Giemsa staining or treating with acridine orange, and reaction with the test substance. By contrast, with the introduction of the fluorescence protein in advance, which makes it possible for the nucleus-related region to emit fluorescent light. Thus, the present invention does not require a further staining step. Therefore, the micronuclei in cells can be detected easily and quickly without having to carry out a fixing treatment such as cytopreparation.
(2) With the conventional technique, it is not possible to detect those micronuclei which are hidden in the macro nucleus as they are located above and underneath the macronucleus region along the measuring direction. By contrast, with the present invention, it is possible to attenuate the fluorescence of only the macronucleus region, or change the fluorescent color. Therefore, all the micronuclei located within the entire region of the cell can be detected. Thus, the present invention makes it possible to carry out the detection of micronuclei in cells with an excellent quantifying property, a high reproducibility, and a very high accuracy.
(3) With present invention, it is possible to detect the creation of a micronucleus in a sequential fashion from the period of the reaction with the chemical substance. Therefore, the background can be eliminated without administering an additional chemical substance such as cytochalasin B, thus improving the efficiency of the method.
(4) Micronuclei can be detected only with light irradiated under certain conditions. Thus, the construction of the optical system is simple, which makes it easy to achieve continuous operation and automation.
(5) With the conventional technique, unevenness or bias of the result may occur when staining the nucleus with reference to the nucleic acid component as an index. By contrast, with use of the nucleus-related protein for the fused gene, the present invention can eliminate such unevenness or bias. Thus, micronuclei can be detected and calculated at a higher quantitative manner.

## Claims

1. A method of detecting a micronucleus in a living cell to be measured, without having to fixate the cell, the method **characterized by** comprising:
generating a mother cell by introducing a fusion gene encoding a single or a plurality of nucleus-related proteins and a fluorescence protein into a culture cell, and making then expressed in the cell;
subjecting the cell to a treatment for a toxicity test or a mutagenicity test of a test substance;
performing a limited excitation which makes a region of the micronucleus emit fluorescence limitedly; and
quantitatively measuring an amount of the fluorescence corresponding to a component of the nucleus-related protein.

2. The detection method according to claim 1, **characterized in that** the nucleus-related protein is of a type specific to a nuclear membrane.

3. The detection method according to claim 1, **characterized by** further comprising:
irradiating light while focusing on a region of a macronucleus of the fused protein expressing mother cell, thereby attenuating the fluorescence of only the macronucleus region, and
detecting the fluorescence derived from only the micronucleus in the cell excluding the macronucleus.
